Europäisches Patentamt

(19) European Patent Office

Office européen des brevets

(11) Numéro de publication: **0 133 827**
**B1**

(12) **FASCICULE DE BREVET EUROPEEN**

(45) Date de publication du fascicule du brevet:
**20.04.88**

(21) Numéro de dépôt: **84401428.2**

(22) Date de dépôt: **05.07.84**

(51) Int. Cl.⁴: **A 61 K 9/36, A 23 P 1/00,
C 09 D 3/18**

(54) Compositions filmogènes pour enrobage des formes solides de produits pharmaceutiques ou alimentaires et produits obtenus revêtus desdites compositions.

(30) Priorité: **06.07.83 FR 8311276**

(43) Date de publication de la demande:
**06.03.85 Bulletin 85/10**

(45) Mention de la délivrance du brevet:
**20.04.88 Bulletin 88/16**

(84) Etats contractants désignés:
**AT BE CH DE FR GB IT LI LU NL SE**

(56) Documents cités:
**DE - A - 2 820 981**

**CHEMICAL ABSTRACTS, vol. 96, no. 14, 5 avril 1982,
page 379, no. 110042e, Columbus, Ohio, US; M.E.
AULTON et al.: "The mechanical properties of
hydroxypropylmethylcellulose films derived from
aqueous systems. Part I: The influence of plasticizers"
DERWENT JAPANESE PATENTS REPORT, vol. S, no. 3,
5 mars 1971, page 5, "Pharmaceuticals", Derwent
Publications Ltd., Londres, GB
CHEMICAL ABSTRACTS, vol. 74, no. 24, 14 juin 1971,
page 323, no. 130342b, Columbus, Ohio, US; J.P.
METRAL-BIOLLAY et al.: "Use of microcrystalline**

(73) Titulaire: **SEPPIC, Société Anonyme dite :, 70, avenue
des Champs Elysées, F-75008 Paris (FR)**

(72) Inventeur: **Brancq, Bernard, 2, rue d'Armenonville,
F-78150 le Chesnay (FR)**
Inventeur: **Malandain, Michel, 52, avenue Amiral
Lemmonier, F-78160 Marly le Roi (FR)**

(74) Mandataire: **Combe, André, CABINET BEAU DE
LOMENIE 55, rue d'Amsterdam, F-75008 Paris (FR)**

(56) Documents cités: (suite)
**cellulose as an adjuvant in the sugar coating of tablets"
POLYMER PROCESSES by Calvin E. SCHILDKNECHT,
Interscience Publishers Inc., N.Y., p. 368-369
Industrial & Engineering Chemistry vol. 54, No. 9, pp.
20-29 (1962)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des compositions filmogènes pour l'enrobage de formes solides, notamment de produits pharmaceutiques, de produits alimentaires ou de semences; elle concerne également le procédé d'enrobage à l'aide desdites compositions et les produits obtenus revêtus desdites compositions.

Les formes sèches pharmaceutiques et certains produits alimentaires, par exemple les comprimés, sont réalisés par agglomération de particules comprenant des excipients et des principes actifs.

Cette agglomération est généralement obtenue par les classiques procédés de granulation, de compactage et de compression.

Afin de protéger les formes sèches contre la dégradation des principes actifs à la lumière ou à l'oxydation, contre l'abrasion lors du conditionnement en blisters, contre la formation de poussières, on recouvre celles-ci d'une pellicule d'un agent d'enrobage.

Cette technique est très connue dans la confiserie ou l'industrie pharmaceutique avec la dragéification au sucre, le pelliculage à la gomme laque en milieu alcoolique dit «gommage» et les vernissages filmogènes vinyliques, acryliques ou cellulosiques. Voir par exemple DE-A 2 820 981.

De même, on a déjà préconisé l'enrobage de graines diverses et notamment de semences; un tel enrobage facilite la manipulation de produits et améliore la tenue de ces semences lorsque celles-ci sont mises en terre.

Ces enrobages isolent les formes sèches et permettent par exemple leur identification par simple coloration de surface.

La dragéification est une opération longue et coûteuse.

Le pelliculage par film réalisé en milieu solvant organique est une technique manuelle ou automatisée plus rapide et économique. Cependant, l'emploi d'alcools ou de solvants chlorés la rend dangereuse.

Depuis quelques années, divers produits et diverses techniques sont proposés pour réaliser des enrobages par film en milieux aqueux. Ces procédés se caractérisent par l'emploi de substances filmogènes hydrosolubles ou hydrodispersibles qui s'appliquent généralement par pulvérisation sur les formes sèches en rotation dans une turbine ou en sustentation dans un lit d'air fluidisé.

L'utilisation d'eau comme solvant ou dispersant des substances filmogènes est économique par rapport aux solvants organiques; cependant, elle constitue une limite sur les points suivants:
- temps d'évaporation allongé,
- concentration faible de l'agent filmogène en solution ou dispersion dans les formulations d'enrobage,
- sensibilité de la forme sèche et du principe actif au milieu aqueux, qui peut provoquer la désintégration de la forme ou la décomposition du principe actif.

Le but de la présente invention est d'éviter les inconvénients précités par la production de nouvelles compositions filmogènes et leur application à l'enrobage des formes solides.

Les compositions filmogènes pour enrobage selon la présente invention sont caractérisées en ce qu'elles contiennent:
- de 15 à 85% en poids d'une substance filmogène cellulosique,
- de 10 à 70% en poids d'au moins une α-cellulose,
- et de 5 à 30% en poids d'un plastifiant alimentaire.

La substance filmogène cellulosique utilisée est un produit connu et précédemment préconisé pour la réalisation d'enrobages. On sait que, parmi les dérivés cellulosiques connus, on peut utiliser les alkyléthers de cellulose, les hydroxyalkyléthers de cellulose, les esters monocarboxyliques de cellulose et les éthersesters mixtes de cellulose. Parmi ces produits, on mentionnera plus spécialement comme dérivés cellulosiques utilisables les hydroxypropylméthylcelluloses et plus particulièrement les hydroxy-propylméthylcelluloses qui présentent une faible viscosité (c'est-à-dire une viscosité de 3 à 15 mPas (cP), à température ambiante, en solution à 2% en poids dans l'eau).

Des teneurs inférieures à 15% en substance filmogène sont insuffisantes pour réaliser un film continu. L'enrobage se présente alors, par examen microscopique, comme une simple juxtaposition de particules.

D'autre part, à des teneurs supérieures à 85% en substance filmogène, l'enrobage réalisé ne diffère pas notablement de ce que l'on réalise dans l'art antérieur.

Avec cette substance filmogène cellulosique, on utilisera un plastifiant alimentaire. Là encore, l'emploi de plastifiants dans les diverses utilisations des substances filmogènes cellulosiques est connu. On sait que les plastifiants utilisables n'ont pas pour fonction essentielle d'abaisser le point de fusion des polymères cellulosiques mais servent à modifier la souplesse et la ténacité des films réalisés avec ces substances cellulosiques.

Le plastifiant requis dans la présente invention peut être un produit hydrophile ou hydrophobe convenablement choisi pour améliorer la souplesse du film réalisé.

Les plastifiants hydrophiles permettent d'obtenir des films rapidement désintégrés en milieu acide ou dans l'eau.

On choisira de préférence un polyol alimentaire ou pharmaceutique du type: glycérine, propylèneglycol, polyéthylèneglycol, sorbitol ou saccharose.

Les plastifiants hydrophobes permettent d'obtenir des films dont la désintégration est retardée ou qui prolongent le masquage de goût.

On choisira ceux-ci parmi les corps gras habituels, tels que les acides, alcools, esters gras et leurs dérivés ou encore les phtalates, citrates ou sébaçates d'éthyle ou de butyle.

La préférence sera donnée à un plastifiant hydrodispersible du type ester de polyéthylèneglycol, par exemple le stéarate de polyoxyéthylène 300, qui facilitera la mise en dispersion de la nouvelle composition filmogène proposée dans le solvant de préparation.

Lesdits plastifiants et leurs diverses fonctions vis-à-vis des propriétés des films obtenus ont déjà été décrits ou suggérés dans diverses publications.

La teneur en plastifiant hydrophile, par exemple le polyéthylèneglycol 6000, est de préférence d'au moins 10% en poids par rapport à la substance filmogène sèche.

La teneur en plastifiant hydrophobe ou hydrodispersible, par exemple le stéarate de polyoxyéthylène (8), est de préférence d'au moins 15% en poids par rapport à la substance filmogène sèche.

Un taux de plastifiant supérieur à 30% dans la nouvelle composition filmogène modifie les propriétés mécaniques de l'enrobage qui devient fragile. Les gravures et barrettes de cassure des comprimés sont alors masquées par un film trop couvrant.

Le troisième constituant essentiel de l'invention est une α-cellulose. On a déjà indiqué que l'α-cellulose était susceptible, grâce notamment à son aptitude à créer des liaisons hydrogènes avec d'autres matériaux, de jouer un rôle de «liant» vis-à-vis par exemple de produits à fonctions OH et notamment de dérivés cellulosiques. On utilisera donc selon la présente invention de 10 à 70% en poids d'α-cellulose dans les mélanges. Par α-cellulose, il faut comprendre les α-celluloses de divers types actuellement connues; on sait par exemple que l'on pourra utiliser selon l'invention les α-celluloses obtenues par broyage mécanique d'α-celluloses naturelles ou les α-celluloses qui ont été obtenues par dépolymérisation partielle (réalisée par hydrolyse acide ou basique) de l'α-cellulose naturelle. De plus, cette α-cellulose joue un rôle important pour réaliser une bonne adhérence du film d'enrobage sur le noyau qui contient généralement (en particulier dans le cas de noyaux pharmaceutiques) des produits tels que de l'amidon, de la cellulose ou des dérivés cellulosiques, des sucres, etc.

Pour obtenir une bonne dispersion de cette α-cellulose dans les mélanges selon l'invention, il est souhaitable que ce produit soit utilisé sous forme d'une poudre fine de granulométrie moyenne inférieure à 100 μm et de préférence inférieure à 50 μm.

Un taux d'α-cellulose inférieur à 10% ne donne pas un pouvoir liant suffisant pour modifier la substance filmogène, et le film n'adhère pas bien au substrat.

Lorsque le taux d'α-cellulose dépasse environ 70% en poids du mélange, on obtient un film dont la souplesse est insuffisante.

Les compositions selon l'invention peuvent comporter en outre les additifs connus et habituellement utilisés pour la modification des propriétés du matériau d'enrobage (couleur, vitesse de dissolution dans divers milieux) et la protection dudit matériau (antioxydant, anti-ultraviolet, ...).

Le procédé préféré pour réaliser un enrobage à l'aide des compositions filmogènes selon la présente invention consiste à dissoudre ou disperser les divers ingrédients du mélange dans un solvant convenable tel qu'un milieu aqueux, puis à pulvériser la solution (ou suspension) obtenue sur des noyaux préalablement préparés.

Les solutions (ou dispersions) aqueuses peuvent atteindre des concentrations allant jusqu'à 25% en poids en composition selon l'invention, ce qui permet de réaliser des enrobages en une durée brève (15 min par exemple).

Les films obtenus sont très couvrants et accrochent fortement au substrat solide.

Ils présentent, de ce fait, une excellente résistance à l'abraison ou au dépelliculage au niveau des arêtes vives, des barrettes de cassure ou des gravures ou des comprimés pharmaceutiques.

La dureté des noyaux enrobés avec ces nouvelles compositions filmogènes est notablement augmentée, ce qui évite leur friabilité.

Le choix de la composition filmogène utilisée et des additifs à cette composition dépendra des applications envisagées.

Ainsi par exemple, grâce à l'utilisation des compositions selon l'invention, il est possible, dans les applications alimentaires ou pharmaceutiques, de modifier ou de masquer le goût de principes actifs amers que les produits peuvent présenter; de plus, il est particulièrement facile de réaliser des formes à libération retardée des principes actifs qu'elles contiennent.

De même, dans le cas de l'enrobage des graines de semences, on peut obtenir une protection efficace contre l'humidité tout en maintenant (ou même en améliorant) les propriétés germinatives desdites semences; on emploiera pour cette application des compositions permettant l'enrobage à température très peu élevée.

Les exemples non limitatifs suivants illustrent l'invention; on rappelle que 1 cP = 1/1000 de PaS, par conséquent, les hydroxypropylméthylcelluloses utilisées dans ces exemples ont une viscosité de 6/1000 de PaS.

Exemple 1

On introduit 500 g d'hydroxypropylméthylcellu-lose (HPMC) qualité 6 mPas (cP) dans un mélangeur Erweka de laboratoire. On y ajoute 2000 g d'α-cellulose pulvérulente de granulométrie 20 μm.

On malaxe le mélange à vitesse lente durant 9 min pour obtenir une masse de poudre homogène.

On mouille ensuite cette poudre par 1000 g de solution aqueuse à 50% de polyéthylèneglycol 6000, en l'ajoutant par petites portions et en maintenant l'agitation mécanique.

La masse humide se transforme en petits agglomérats compacts ou granulés après un temps de mélange de 10 à 15 min. Ces agglomérats sont calibrés sur une grille oscillante Frewitt.

Ces agglomérats sont déposés sur des tamis de séchage dans un four ventilé porté à 60°C jusqu'à évaporation de l'humidité.

On récupère alors 2955 g de granulés secs, appelés produit A, ayant une teneur en eau résiduelle de 4 à 5% maximum.

Ces granulés sont tamisés pour conserver la fraction 20 à 200 μm, les fines étant recyclées dans le mélangeur.

Une série de tests est conduite sur le produit A pour mesurer ses propriétés filmogènes.

a) Mise en dispersion.

On prépare une dispersion (1) de 220 g du produit A dans 780 g d'eau à 40°C par agitation mécanique lente (500 tr/min) à l'aide d'un RAYNERI de laboratoire. On obtient une dispersion fine et homogène en 15 min.

Par comparaison, une dispersion (2) de 100 g d'hydroxypropylméthylcellulose 6 mPas (cP) dans 900 g d'eau à 40°C par le même système et à la même vitesse demande plus de 30 min. La dispersion présente des grumeaux et il est nécessaire de disperser ces amas qui augmentent la vitesse d'agitation à plus de 2000 tr/min pour homogénéiser la dispersion (2).

b) Caractéristiques de la dispersion.

La dispersion (1) du produit A se présente sous une forme de liquide peu visqueux, s'écoulant librement et blanchâtre.

La teneur en matière sèche est de 21,8% (3 h à 105°C).

L'aspect microscopique de la dispersion (1) présente des particules colloïdales.

L'étalement de la dispersion (1) à la jauge micromètre 50 μm sur une plaque de verre montre l'absence de particules solides à l'œil nu.

c) Propriétés filmogènes du produit A.

La dispersion (1) se prête aisément à la pulvérisation avec un pistolet du type «Airspray» de marque Binks, modèle 960 équipé d'une buse de 1 mm et alimenté avec une pression d'air de 3,5 kg.

On forme un film mince du produit A sur une plaque de verre par pulvérisation de la solution (1) sur celle-ci jusqu'à obtenir une épaisseur de 20 μm après séchage de la plaque à l'air à 60°C.

Ce film est translucide et se décolle aisément de la plaque de verre. Il est souple et non cassant.

Exemple 2

a) Composition.

Hydroxypropylméthylcellulose (6 mPas (CP)) (HPMC) 42
Cellulose alpha (20 μm) 35
PEG 400 10
$TiO_2$ – oxyde de titane 8
Laque aluminique de colorant Erythrosine 5

b) Préparation.

– Introduire dans un granulateur Lödige de 50 l 8,2 kg d'hydroxypropylméthylcellulose 6 mPas (cP) et 7 kg de cellulose microcristalline de 20 μm.

– Préparer le liquide de mouillage comprenant 2 l d'eau, 2 kg de PEG 400, 1,6 kg d'oxyde de titane, 1 kg de laque aluminique Erythrosine et 2 kg de solution aqueuse à 10% d'hydroxypropylméthyl-cellulose 6 mPas (cP), soit 200 g d'hydroxypropylméthylcellulose.

– Le liquide de mouillage est mélangé sur disperseur rapide, puis broyé.

– Le liquide de mouillage est ajouté lentement dans le mélangeur sur le mélange de poudre en mouvement.

– Il se forme un grain régulier rose.

– Le grain humide est calibré sur grille Frewitt de 1 mm, séché sur lit d'air fluidisé puis tamisé à nouveau.

– La poudre granuleuse obtenue a une densité de 0,5 environ. Elle est de couleur rose uniforme.

c) Utilisation.

– Disposer 200 g de cette préparation dans 800 g d'eau froide; une dispersion homogène est obtenue après 20 min environ.

– Cette préparation permet l'enrobage en 25 min de 10 kg de comprimés dans une turbine à ventilation forcée.

Exemple 3

– On introduit dans un mélangeur granulateur DIOSNA:

20 kg d'hydroxypropylméthylcellulose 6 mPas (cP),

16 kg de cellulose microcristalline de granulométrie moyenne 20 μm.

– On mélange les poudres pendant 5 min.

– D'autre part, on prépare 20 l d'une dispersion aqueuse D composée de 4 kg de stéarate de polyoxyéthylène 8 et de 16 l d'eau. La dispersion est effectuée à 45°C.

La dispersion aqueuse D est introduite progressivement dans le mélangeur en rotation. Après la fin de l'introduction, la granulation est poursuivie pendant 7 min.

– Le granulé ainsi obtenue est de dimension granulométrique régulière comprise à 95% entre 100 et 1000 μm.

– Le granulé est séché sur étuve à plateau jusqu'à une humidité résiduelle de 2%.

– Le granulé G ainsi obtenu se conserve parfaitement.

– Il se disperse aisément à température ambiante dans l'eau, dans les mélanges eau-alcool à la condition que ceux-ci aient une teneur minimale de 15% d'eau, ainsi que dans les mélanges alcools-solvants chlorés (dichlorométhane par exemple). La dispersion totale à une concentration de 15% à 20% est obtenue sous agitation faible en 20 min dans l'eau et les mélanges eau-alcool (méthylique, éthylique ou isopropylique).

– Dans les mêmes conditions, les hydroxypropylméthyl-celluloses telles que le Méthocel $B_5$ (marque déposée) nécessitent une agitation violente.

– On provoque la formation de grumeaux difficiles à dissoudre par la suite ou nécessitant un temps de repos de 12 à 24 h.

Exemple 4 (UTILISATION INDUSTRIELLE)

Ⓐ – Equipement:
turbine Manesty Accelacota

pistolets 4 Walter WA 15.
avec buses de pulvérisation de diamètre 1 mm.

Comprimés:
220 kg de placebo gravés: Lactose Fast Flo (marque déposée par Foremost – USA)/Avicel(marque déposée par FMC – USA)/stéarate de magnésium 49,7/49,7/0,6 de dureté 10 kg.
– On prépare une dispersion d'enrobage composée de la façon suivante: 3,2 kg du granulé obtenu à l'exemple 3 sont introduits dans 14,4 l d'eau sous agitation modérée. L'agitation est maintenue pendant 25 min. On ajoute à cette dispersion 2,4 kg de SEPISPERSE AP 3012 (marque déposée).

Suspension pigmentaire d'oxyde de fer jaune et d'oxyde de titane en milieu eau-propylèneglycol, l'agent de suspension étant constitué de HPMC 6 mPas (cP).

Caractéristiques de la suspension pigmentaire:

| | |
|---|---|
| teneur en pigment | 35% |
| teneur en HPMC | 2% |
| teneur en propylèneglycol | 30% |

Après mélange, la dispersion d'enrobage est prête à l'emploi. Sa viscosité est de 800 mPas (cP). Sa teneur en matières sèches est de 20,4%.

Paramètres opératoires
1. Préchauffage des comprimés à 40°C
2. Pendant 3 min:

| | |
|---|---|
| rotation de la turbine | 3 tr/min |
| température d'entrée d'air | 80°C |
| débit de pulvérisation | 250 g/min/pistolet |
| pression de pulvérisation | 4 kg/cm$^2$ |

3. Pendant 20 min:

| | |
|---|---|
| rotation de la turbine | 6 tr/min |
| température d'entrée d'air | 80°C |
| débit de pulvérisation | 170 g/min/pistolet |
| pression de pulvérisation | 4 kg/cm$^2$ |

– Quantité totale pulvérisée: + 16,6 kg, soit en produit sec: 16,6 × 20,4% = 3,38 kg.
– Gain de poids moyen des comprimés: 1,5%.
– Enrobage régulier d'aspect satiné.
– Temps total de pulvérisation: 23 min.

Ⓑ – A titre comparatif, on réalise dans les mêmes conditions d'équipement, de charge et de comprimés, une formulation classique à base de HPMC 6 mPas (Cp).

On introduit sous agitation violente 3,2 kg de Pharmacoat 606 dans 29 l d'eau. On maintient l'agitation violente pendant 30 min, puis on laisse reposer la solution pendant 12 h. On introduit ensuite dans cette dispersion 2,4 kg de SEPISPERSE AP 3012; on homogénéise. La viscosité de la dispersion d'enrobage, prête à l'emploi est de 800 mPas (cP) indique à l'essai précédent pour une teneur en matière solide dans la dispersion de: 11,5%.

Paramètres opératoires
1. Préchauffage des comprimés à 40°C.

2. Pendant 50 min:

| | |
|---|---|
| rotation de la turbine | 5 tr/min |
| température d'entrée d'air | 150°C |
| débit de pulvérisation | 4 g/min/pistolet |
| pression de pulvérisation | 4 kg/cm$^2$ |

Il n'est pas possible d'accroître le débit de liquide car risque de collage.
– Quantité pulvérisée: 600 g × 50 = 30 kg, soit en produit sec: 3,45 kg.
– Gain de poids moyen des comprimés: 1,5%.
– Enrobage régulier d'aspect légèrement plus brillant que dans l'exemple précédent.
– Gain de temps de pulvérisation: 54%.
– Gain de temps sur l'opération complète: 35%.

Exemple 5
– Le granulé G obtenu dans l'exemple 3 est utilisé pour l'enrobage de comprimés d'hydrolysat de pancréas dans les conditions suivantes:
Comprimés de 400 mg composés d'extrait de pancréas du porc (250 mg) et d'excipients (Lactose Fast Flo/Avicel). Ils sont obtenus par la technique de double compression et ont une dureté de 3 kg.
Quantité de comprimés: 25 kg.
Dispersions d'enrobage:

| | |
|---|---|
| granulé G | 600 g |
| Sepisperse° K 3011 | 400 g |
| Ethanol 95 | 3000 g |
| Eau | 750 g |

On introduit 600 g de granulé G dans un mélange eau-éthanol composé de 750 g d'eau et 3000 g d'éthanol à 95°C sous agitation. L'agitation est maintenue pendant 17 min, puis on ajoute 400 g d'une dispersion pigmentaire en milieu alcoolique d'une teneur en solide de 45% et on homogénéise le mélange.
Matériel:
Turbine classique à dragéifier de 40 kg de capacité.
2 pistolets Airspray Binks 460.
Paramètres opératoires:
Pas de préchauffage.
Pression de pulvérisation: 3 kg/cm$^2$.
Débit de liquide: 40 g/min/pistolet.
Durée: 1 h.
Température des comprimés maintenue entre 25 et 30°C.

Résultats
– Enrobage régulier, d'aspect satiné
– Gain de poids des comprimés: 2,5%
– Dureté après enrobage: 10 kg
soit un accroissement de 7 kg par rapport aux comprimés nus.

**Revendications**

1. Compositions filmogènes destinées à l'enrobage des formes solides telles que produits pharmaceutiques ou alimentaires ou semences, caractérisées en ce qu'elles comportent en poids:

– 15 à 85% d'une substance filmogène cellulosique,
– 10 à 70% d'au moins une α-cellulose,
– 5 à 30% d'au moins un plastifiant alimentaire.

2. Compositions selon la revendication 1, caractérisées en ce que ladite substance filmogène cellulosique est choisie parmi les alkyléthers de cellulose, les hydroxyalkyléthers de cellulose, les esters monocarboxyliques de cellulose et les éthers-esters mixtes de cellulose.

3. Compositions selon la revendication 2, caractérisées en ce que ladite substance filmogène cellulosique est une hydroxypropylméthylcellulose de faible viscosité.

4. Compositions selon l'une des revendications 1 à 3, caractérisées en ce que l'α-cellulose est utilisée sous forme d'une poudre de granulométrie inférieure à 100 μm et de préférence inférieure à 50 μm.

5. Compositions selon l'une des revendications 1 à 4, caractérisées en ce que le plastifiant est choisi parmi le polyéthylèneglycol et le stéarate de polyoxyéthylène.

6. Procédé pour la mise en œuvre des compositions selon l'une quelconque des revendications 1 à 5, caractérisé en ce que l'on réalise une solution ou dispersion, en milieu aqueux de préférence des ingrédients desdites compositions et que l'on pulvérise ladite préparation sur les noyaux à enrober.

7. Produits enrobés à usage alimentaire ou pharmaceutique, caractérisés en ce que l'enrobage a été réalisé à l'aide d'une composition selon l'une des revendications 1 à 5.

## Patentansprüche

1. Filmbildende Zusammensetzungen zur Umhüllung von festen Formen, wie pharmazeutischen oder Ernährungsprodukten oder Samen, dadurch gekennzeichnet, dass sie gewichtsmässig umfassen:
– 15 bis 85% einer filmbildenden Zellulosesubstanz,
– 10 bis 70% mindestens einer α-Zellulose,
– 5 bis 30% mindestens eines Speiseplastifizierungsmittels.

2. Zusammensetzungen nach Anspruch 1, dadurch gekennzeichnet, dass die filmbildende Zellulosesubstanz ausgewählt ist aus Zellulosealkylethern, Zellulosehydroxyalkyethern, Zellulosemonocarboxylestern und gemischten Zelluloseetherestern.

3. Zusammensetzungen nach Anspruch 2, dadurch gekennzeichnet, dass die filmbildende Zellulosesubstanz Hydroxypropylmethylzellulose geringer Viskosität ist.

4. Zusammensetzungen nach einem der Ansprüche 1 bis 3, dadurch gekennzeichnet, dass die α-Zellulose in Form eines Pulvers mit einer Korngrösse von unter 100 μm, vorzugsweise unter 50 μm, verwendet wird.

5. Zusammensetzungen nach einem der Ansprüche 1 bis 4, dadurch gekennzeichnet, dass das Plastifizierungsmittel ausgewählt ist aus Polyethylenglykol und Polyoxyethylenstearat.

6. Verfahren zur Anwendung der Zusammensetzungen nach einem der Ansprüche 1 bis 5, dadurch gekennzeichnet, dass man eine Lösung oder Dispersion der Ingredienzien der Zusammensetzungen, vorzugsweise in wässerigem Milieu herstellt und die Präparation über die zu umhüllenden Kerne zerstäubt.

7. Umhüllte Produkte zur Verwendung in der Ernährung oder Pharmazie, dadurch gekennzeichnet, dass die Umhüllung mit Hilfe einer Zusammensetzung nach einem der Ansprüche 1 bis 5 realisiert worden ist.

## Claims

1. Film-forming compositions for enveloping solid forms such as pharmaceutical or food products or seeds, characterized in that they comprise, by weight:
– 15 to 85% of a cellulosic film-forming substance,
– 10 to 70% of at least one alpha-cellulose,
– 5 to 30% of at least one plasticizer suitable for consumption.

2. Compositions according to claim 1, characterized in that said cellulosic film-forming substance is selected from the alkylethers of cellulose, the hydroxyalkylethers of cellulose, the monocarboxylic esters of cellulose and the mixed ether esters of cellulose.

3. Compositions according to claim 2, characterized in that said cellulosic film-forming substance is a hydroxypropylmethylcellulose of low viscosity.

4. Compositions according to one of claims 1 to 3, characterized in that the alpha-cellulose is used in the form of a powder with a granulometry of less then 100 μm, and preferably of less than 50 μm.

5. Compositions according to one of claims 1 to 4, characterized in that the plasticizer is selected from polyethyleneglycol and the stearate of polyoxyethylene.

6. Process for using the compositions according to any one of claims 1 to 5, characterized in that a solution or dispersion is made, preferably in an aqueous medium, of the ingredients of said compositions, and said preparation is sprayed on the cores to be enveloped.

7. Enveloped products for food or pharmaceutical use, characterized in that the composition according to one of claims 1 to 5 was used for enveloping.